# EUROPEAN PATENT SPECIFICATION

(11) **EP 2 027 145 B1**
(45) Date of publication and mention of the grant of the patent: **07.03.2012**
(21) Application number: 07725981.0
(22) Date of filing: 12.06.2007
(51) Int. Cl.: C07K 14/00, C08G 69/10, C07K 1/00, A61K 47/48

(54) **A PROCESS FOR THE PREPARATION OF POLY-ALFA-GLUTAMIC ACID AND DERIVATIVES THEREOF**
VERFAHREN ZUR HERSTELLUNG VON POLY-A-GLUTAMINSÄURE UND DERIVATEN DAVON
PROCÉDÉ DE PRÉPARATION D'ACIDE ALPHA POLYGLUTAMIQUE ET DÉRIVÉ CELLE-CI

(30) Priority: 15.06.2006 EP 06012351; 15.06.2006 US 813787 P
(43) Date of publication of application: 25.02.2009
(60) Divisional application: 11162831.9
(73) Proprietor: Cell Therapeutics, Inc. - Sede Secondaria, 20091 Bresso (IT); CELL THERAPEUTICS, INC., Seattle, WA 98119 (US)
(72) Inventor: MCKENNON, Marc, Seattle, WA 98119 (US); DA RE, Giovanni, 20139 Milano (IT); FELICIOTTI, Luca, 20099 Sesto San Giovanni (IT); ARTICO, Marco, 20015 Parabiago (IT); PARDI, Gianluca, 56010 Ripafratta (IT); GRUGNI, Mario, 20026 Novate (IT)
(74) Representative: Minoja, Fabrizio
(86) International application number: PCT/EP2007/005172
(87) International publication number: WO 2007/144140

(56) References cited:
- WO-A-02/34237
- WO-A-99/64495
- WO-A2-01/94379
- US-A- 3 635 909
- GALLOT B ET AL: "Synthesis and structural study by X-ray diffraction of lyotropic block lipopeptidic polymers with polylysine and poly(glutamic acid) peptidic chains" MOLECULAR CRYSTALS AND LIQUID CRYSTALS UK, vol. 153, December 1987 (1987-12), pages 347-356, XP009074726 ISSN: 0026-8941
- SHIH I-L ET AL: "BIOMEDICAL APPLICATIONS OF CHEMICALLY AND MICROBIOLOGICALLY SYNTHESIZED POLY(GLUTAMIC ACID) AND POLY(LYSINE)" MINI REVIEWS IN MEDICINAL CHEMISTRY, BENTHAM SCIENCE PUBLISHERS, HILVERSUM, NL, vol. 4, no. 2, 2004, pages 179-188, XP008034061 ISSN: 1389-5575
- DEMING T J: "FACILE SYNTHESIS OF BLOCK COPOLYPEPTIDES OF DEFINED ARCHITECTURE" NATURE, NATURE PUBLISHING GROUP, LONDON, GB, vol. 390, 27 November 1997 (1997-11-27), pages 386-389, XP000918865 ISSN: 0028-0836
- WILCHEK M ET AL: "New synthesis of poly-L-glutamic acid and poly-L-glutamyl proteins." ARCHIVES OF BIOCHEMISTRY AND BIOPHYSICS. MAR 1966, vol. 113, no. 3, March 1966 (1966-03), pages 742-749, XP009074728 ISSN: 0003-9861
- RINAUDO & DOMARD: BIOPOLYMERS, vol. 14, 1975, pages 2035-2048, XP002423281
- UEHARA T ET AL: "Two [beta]-forms and the [alpha]-helix of N-octanoyl-L-glutamic acid oligomers" JOURNAL OF THE CHEMICAL SOCIETY FARADAY TRANSACTIONS UK, vol. 88, no. 23, 7 December 1992 (1992-12-07), pages 3451-3459, XP009080140 ISSN: 0956-5000

## Description

### Field of the invention

The present invention relates to a process for the preparation of poly-α-glutamic acid and derivatives thereof.

### Background of the invention

### Poly-α-glutamic acid of formula (I)

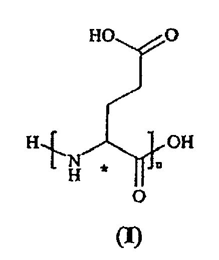

is currently used as drug delivery system, for example for the preparation of conjugates with a wide variety of drugs containing a primary or secondary amino group as well as primary, secondary or tertiary hydroxy groups. The conjugation can occur directly or through a suitable linker, for example an amino acid or a hydroxyacid. See, for example, EP0932399, Advanced Drug Delivery Reviews, Volume 54, Issue 5, 2002, Pages 695-713 and Journal of Controlled Release, Volume 74, Issues 1-3, 2001, Pages 243-247.

Poly-α-glutamic acid can be synthesized by polymerisation of suitably protected glutamic acid. US 3,635,909 discloses, *inter alia*, the polymerisation of D-glutamic acid-γ-(*tert*-butyl)ester N-carboxy anhydride in a 1,2-dichloroethane/1,4-dioxane mixture using sodium 4-methyl-2-pyrrolidone as initiator.

Several methods for the preparation of poly-γ-benzyl-α-glutamic acid are described in WO01/94379 A2. Removal of the benzyl protective group from the polymers is performed with hydrobromic acid, to give poly-α-glutamic acids.

The preparation of poly-α-glutamic acid by polymerisation of the N-carboxy anhydride of γ-benzyl-L-glutamic acid using octadecylamine as the initiator is described in Mol. Cryst. Liq. Cryst., 1987, Vol 153, pp. 347-356. The obtained protected poly-glutamic acid is deprotected using basic hydrolysis. The obtained polymer contains a maximum of 20 aminoacids and bears a lipophilic C 18 chain at the C-terminus, originating from the initiator.

The preparation of N-terminally functionalised poly-α-aminoacids using polymerisation of the N-carboxy anhydrides of aminoacids is disclosed in WO99/64495 A.

### Description of the invention

The present disclosure shows that appropriate choice of the solvent and of the initiator allows the control of the molecular weight of poly-α-glutamic acid prepared from tertiary γ-esters of α-glutamic acid N-carboxy anhydride. Accordingly, the invention relates to a process for the preparation of poly-α-glutamic acid (I) 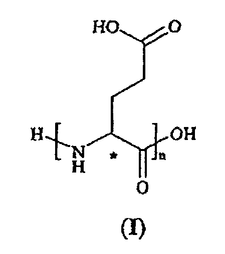 wherein n is an integer comprised between 60 and 310, so that the poly-α-glutamic acid has a molecular weight ranging from 8,000 to 40,000 Da, preferably from 10,000 to 35,000 Da, more preferably from 13,000 to 16,000 Da, and with a polydispersity index usually ≤2, preferably ≤1.5,
said process comprising:
a. the polymerisation of a tertiary γ-ester of α-glutamic acid N-carboxy anhydride of formula (**II**) 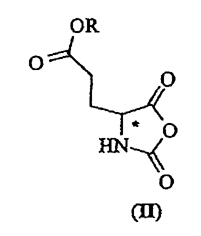 wherein R is selected from t-butyl, 1,1-dimethylpropyl- and 1,1-dimethylbutyl- and is preferably a t-butyl group
   either in water or in an organic solvent selected from tetrahydrofuran (THF), 1,4-dioxane, dimethylformamide (DMF), 1,4-dioxane/DMF and 1,4-dioxane/tetrahydrofuran mixtures with an initiator selected from
   potassium *tert*-butoxide (t-BuOK), sodium methoxide (MeONa), diisopropylethylamine, 1,8-diazabicyclo[5,4,0]undec-7-ene (DBU), 4-dimethylaminopyridine (DMAP), glutamic acid dimethyl ester, and glutamic acid-γ-*tert*-butyl ester, to give a compound of formula (**III**) 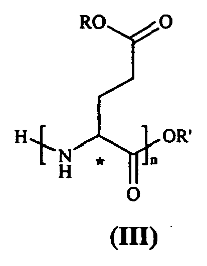 wherein n and R are as defined above and R' is hydrogen when the initiator is selected from diisopropylethylamine, 1,8-diazabicyclo[5,4,0]undec-7-ene (DBU), 4-dimethylaminopyridine (DMAP), glutamic acid dimethyl ester and glutamic acid-γ-*tert*-butyl ester or a *t*-butyl or methyl group when the initiator is t-BuOK and MeONa respectively followed by
b. acid hydrolysis of the ester groups.

Throughout the specification, the symbol * indicates a chiral center and the term glutamic acid comprises both the L- and D- form, either as pure isomers or as racemic mixture.

Step a) is usually carried out at a temperature ranging from 10 to 50°C, with a concentration of the γ-ester of α-glutamic acid N-carboxy anhydride ranging from 0.1 to 0.3 M. According to a preferred embodiment, the process is carried out using 1,4-dioxane as the solvent and DBU as the initiator.

The molar ratio of γ-ester of α-glutamic acid N-carboxy anhydride to initiator usually ranges from 2 to 25; the molar ratio of γ-ester of α-glutamic acid N-carboxy anhydride to α-glutamic acid γ-ester is 100.

Step b) can be carried out using any reagent conventionally used for the removal of *tert*-butyl esters, as exemplified by those described in "Protective groups in organic synthesis" Third edition, Theodora W. Greene and Peter G. M. Wuts, a Wiley-Interscience publication- John Wiley & Sons, Inc, page 406-408 and references therein. Preferred conditions include trifluoroacetic acid (TFA), formic acid and water/formic acid mixtures at a temperature ranging from 20 to 60°C; TFA is used in amount of 100 v/w, while formic acid and water/formic acid mixtures are used in amounts ranging from 20 to 50 v/w.

The process of the invention is suitable for preparing both the D and L form of poly-α-glutamic acids, in particular the L form. Moreover, the poly-α-glutamic acids prepared according to the invention can be easily converted into their acidic or basic salts by reaction with inorganic acids, such as hydrochloric acid, hydrobromic acid, sulphuric acid, nitric acid, perchloric acid or with inorganic bases such as alkaline or alkaline-earth metal hydroxides and ammonium hydroxide.

The process of the invention provides poly-α-glutamic acid with a weight average molecular weight (M_{w}) determined by Gel Permeation Chromatography combined with Multi Angle Laser Light Scattering detection, ranging within the values defined above, preferably between 13,000 and 16,000 Da and with a polydispersity index ≤ 1.5. This is particularly advantageous, since the procedures disclosed in the literature, through different γ-protecting groups (such as benzyl, methyl or ethyl; see Polymer monographs, Volume 9: H. Block, Poly(γ-benzyl-L-glutamate) and other glutamic acid containing polymers. Edited by B. Huglin, University of Salford), afford materials of uncontrolled and very high molecular weight, which often require chromatographic separation to isolate poly-α-glutamic acids having a desidered molecular weight range.

Moreover, the process of the invention allows to avoid spontaneous formation of a pyroglutamic ester at the amino terminus, which usually occurs during polymerisation using, for example, γ-benzyl, γ-methyl or γ-ethyl glutamic acid ester N-carboxy anhydride. In fact, currently available poly-α-glutamic acids exhibit either a pyroglutamic terminus, or an amino terminus or a mixture thereof. Suppression of formation of a pyroglutamic terminus is achieved in particular when a compound of formula (II) wherein R is a t-butyl group is used as the starting material. Poly-α-glutamic acids γ-esters with a free amino terminus can thus be reacted either with stoichiometric amounts of pyroglutamic acid or with other suitable acids, so as to achieve 100% conversion into the desired N-capped poly-α-glutamic acid.

The invention will be hereinafter illustrated in greater detail in the following examples.

### EXAMPLE 1

### Step a - Preparation of L-α-Glu-NCA-γ-tBu ester

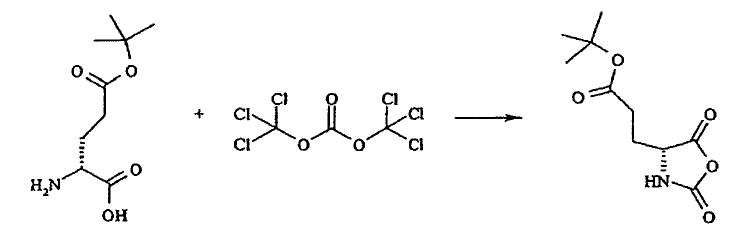

40.17 g of triphosgene (0.135 mol; phosgene/ L-Glu-γ-tBu ester 2.74/1) and 862.5 mL of THF were placed under nitrogen into a 1 L jacketed reactor, equipped with mechanical stirrer. The reactor was thermostated at 20°C and the mixture was stirred until complete dissolution of triphosgene (a few minutes are necessary), thereafter 30 g of L-α-Glu-γ-tBu ester (0.148 mol) were added in a single portion and quite rapidly. The resulting suspension was allowed to react for 2 hours at 20°C. At the beginning exothermicity was observed (≈ 4°C), then the solid dissolved as the reaction proceeded: after about 30 min a clear solution was obtained.

At the end of the reaction the solvent was evaporated under reduced pressure, keeping the jacket temperature not higher than 20°C: the distillation was quite tumultuous. The distillation was continued until a dense and oily residue was obtained, without solids (about 80 mL of residue expected). At the end of the distillation 750 mL of n-heptane were dropped in over about 15 min and the mixture was stirred under nitrogen at 20/23 °C for 1 hour until complete crystallization of the product. Usually, crystallisation begins after addition of 150 mL of n-heptane.

The resulting pure white solid was filtered on buchner funnel and washed with 3 × 90 mL of n-heptane, then dried in the oven under dynamic vacuum at 20/25 °C for no longer than 20 hours. The solid must be used as soon as possibile and must be stored under static vacuum in the presence of silica gel.

Starting from 30 g of L-α-Glu-γ-tBu ester, 23.3 g of L-α-Glu-NCA-γ-tBu ester were obtained (68.8 % yield).

### Step b - Polymerization of α-L-glutamic acid-γ-(tert-butyl)ester N-carboxy anhydride) (NCA) to poly-α-L-glutamic acid-γ-(tert-butyl)ester.

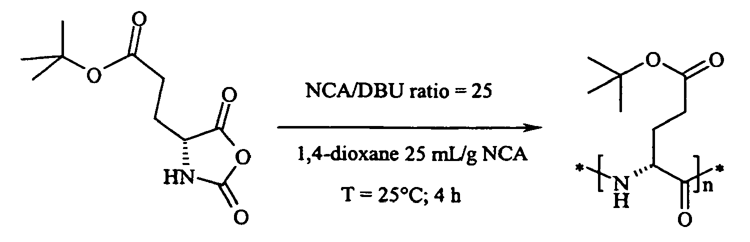

1,8-Diazabiciclo[5.4.0]undec-7-ene free base (DBU; MW 152.24; 585 mg, 3.84 mmol) was dissolved in 1,4-dioxane (5 mL from the total amount) in few minutes.

In a 1 L jacketed glass reactor 1,4-dioxane (550 mL) and α-L-glutamic acid-γ-(*tert*-butyl)ester N-carboxy anhydride (NCA) (22 g, 96 mmol; NCA/DBU = 25) were mixed at 25°C until dissolution was complete.

The resulting solution was added very quickly with the previously prepared DBU solution. A precipitate instantly appeared and low exothermicity was observed (about 3°C).

The mixture was stirred for 4 hours, then water (1100 mL) was slowly poured in over 30 to 40 minutes. After a further 30 minutes stirring at 25°C the precipitated solid was collected by filtration and washed with water (3 x 50 mL). The recovered white solid was then dried under vacuum at 40°C to yield 16.4 g of pure material, t-BuPG (92% recovery; DPₙ= 137.2, number average degree of polymerisation; Mₙ = 25,400 Da, number average molecular weight from ¹H-NMR).

### Step c - Hydrolysis of poly-α-L-glutamic acid-γ-(tert-butyl)ester to poly-α-L-glutamic acid

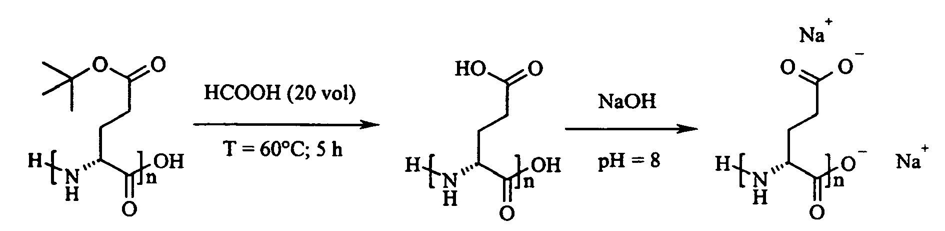

Solid poly-α-L-glutamic acid-γ-(*tert*-butyl)ester (4 g) was suspended in formic acid 99% (80 mL, 20 volumes) under nitrogen atmosphere in a 100 mL jacketed glass reactor. The mixture was stirred at 60°C for 5 hours. After about a 30-minute heating the suspension turned into solution, then a solid began to precipitate.

Most of the formic acid was then removed by distillation under reduced pressure (P = 15 torr; internal temperature always below 60°C). The residual formic acid was evaporated off by azeotropic distillation with toluene (2 x 40 mL; T<40°C).

The resulting residue was suspended in water (40 mL) and the mixture was cooled to 3 - 5°C, then 30% w/w NaOH was carefully added to reach pH 8. Once the pH was stable at 23 - 25°C, the resulting solution was filtered through a 0.22 µm microfilter.

The clear solution was acidified by means of sulphuric acid up to pH 2.5, then the resulting suspension was stirred for 2 hours. The precipitated solid was filtered off and dried under vacuum (30°C overnight). A white cake was recovered (2.7 g) with a M_{w} = 13,900 Da and polydispersity 1.04 (GPC - MALLS).

### EXAMPLE 2

α-L-glutamic acid-γ-(*tert*-butyl)ester N-carboxy anhydride (3.95 g, 17.2 mmol) was dissolved, under nitrogen stream, in THF (100 mL) to reach a concentration of 0.039 g/mL (0.17 M). The stirring speed was set to 800 rpm.

Then DBU (105 mg, A/I=25) was added by a syringe very quickly and the mixture was stirred for 4 hours at 22-23°C. A slight exotherm was observed soon after addition of the initiator.

Afterwards, the reaction mixture was concentrated to dryness and the residue was rinsed with fresh 1,4-dioxane (100 mL). Two volumes of water were then added to the reaction mixture which was stirred for 30 minutes. The precipitated solid was collected, washed with water and dried under vacuum at 40°C for at least 12 hours.

2.85 g of poly-α-L-glutamic acid-γ-(*tert*-butyl)ester (90% recovery) as a white powder with Mₙ = 34,100 Da and DPₙ = 184 (by ¹H-NMR) were obtained.

The resulting poly-α-L-glutamic acid-γ-(*tert*-butyl)ester was then hydrolysed in hot 99% HCOOH according to the procedure described in step c of Example 1, furnishing about 1.7 g of poly-α-L-glutamic acid (84% recovery) with M_{w} = 25,700 and polydispersity = 1.2, n ≈ 100 according to Gel Permeation Chromathography coupled with Multi Angle Laser Light Scattering analysis.

## Claims

1. A process for the preparation of poly-α-glutamic acid of formula (I) 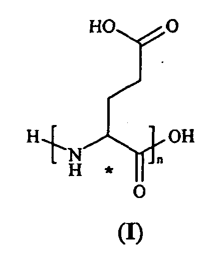 wherein the symbol * indicates a chiral center n is between 60 and 310, so that the poly-α-glutamic acid has a molecular weight ranging from 8,000 to 40,000 Da
said process comprising the steps of:
a) polymerisation of a tertiary γ-ester of α-glutamic acid N-carboxy anhydride of formula (II) 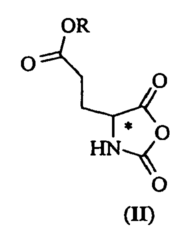 wherein the symbol * is as defined above and R is selected from *t*-butyl, 1,1-dimethylpropyl and 1,1-dimethylbutyl
in water or in an organic solvent selected from: tetrahydrofuran, 1,4-dioxane, dimethylformamide, 1,4-dioxane/DMF and 1,4-dioxane/tetrahydrofuran mixture with an initiator selected from potassium *tert*-butoxide, sodium methoxide, diisopropylethylamine, 1,8-diazabicyclo[5,4,0]undec-7-ene, dimethylaminopyridine and L-glutamic acid-γ-*tert*-butylester, to give a compound of formula (**III**) 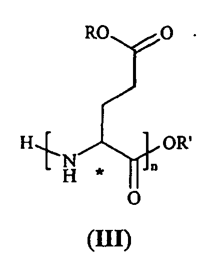 wherein * and R are as defined above and R' is hydrogen when the initiator is selected from diisopropylethylamine, 1,8-diazabicyclo[5,4,0]undec-7-ene, 4-dimethylaminopyridine, glutamic acid dimethyl ester and glutamic acid-γ-*tert*-butyl ester or R' is a *t*-butyl or methyl group when the initiator is potassium *tert*-butoxide and sodium methoxide respectively and
followed by
b) acid hydrolysis of the γ- and α-ester groups to give a compound of formula (**I**).

2. The process according to claim 1 wherein the solvent is 1,4-dioxane and the initiator is 1,8-diazabicyclo[5,4,0]undec-7-ene.

3. The process according to claim 1 or 2 wherein the temperature ranges from 10 to 50°C.

4. The process according to any one of claims 1 to 3 wherein the concentration of the tertiary γ-ester of α-glutamic acid N-carboxy anhydride ranges from 0.1 to 0.3 M.

5. The process according to any one of claims 1 to 4 wherein step b) is carried out in an acid selected from trifluoroacetic acid, formic acid and water/formic acid mixtures at a temperature ranging from 20 to 60°C.

6. The process according to any one of claims 1 to 5 wherein R is t-butyl.

7. The process according to any one of claims 1 to 6 wherein the molecular weight of the poly-α-glutamic acid (I) ranges from 13,000 to 16,000 Da and the polydispersity index is ≤ 1.5.

## Patentansprüche

1. Verfahren zur Herstellung von Poly-α-glutaminsäure der Formel (I) 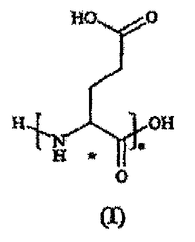 worin das Symbol * ein chirales Zentrum bezeichnet, n zwischen 60 und 310 liegt, sodass die Poly-α-glutaminsäure ein Molekulargewicht im Bereich von 8.000 bis 40.000 Da hat,
wobei das Verfahren folgende Stufen umfasst:
a) Polymerisation eines tertiären γ-Esters des α-Glutaminsäure-N-carboxyanhydrids der Formel (II) 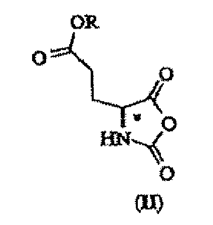 worin das Symbol * wie oben definiert ist und R ausgewählt ist aus t-Butyl, 1,1-Dimethylpropyl und 1,1-Dimethylbutyl,
in Wasser oder einem organischen Lösungsmittel, ausgewählt aus: Tetrahydrofuran, 1,4-Dioxan, Dimethylformamid, 1,4-Dioxan/DMF und 1,4-Dioxan/Tetrahydrofuran, gemischt mit einem Initiator, ausgewählt aus Kalium-tert-butoxid, Natriummethoxid, Diisopropylethylamin, 1,8-Diazabicyclo[5,4,0]undec-7-en, Dimethylaminopyridin und L-Glutaminsäure-γ-tert-butylester, um eine Verbindung der Formel (III) zu ergeben 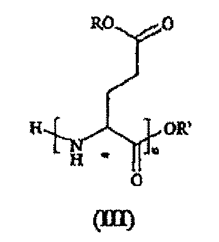 worin * und R wie oben definiert sind und R' Wasserstoff ist, wenn der Initiator ausgewählt ist aus Diisopropylethylamin, 1,8-Diazabicyclo[5,4,0]undec-7-en, 4-Dimethylaminopyridin, Glutaminsäuredimethylester und Glutaminsäure-γ-*tert*-butylester, oder R' eine t-Butyl oder eine Methylgruppe ist, wenn der Initiator Kalium-tert-butoxid bzw. Natriummethoxid ist, und
gefolgt von
b) Säurehydrolyse der γ- und α-Estergruppen, um eine Verbindung der Formel (I) zu ergeben.

2. Verfahren nach Anspruch 1, wobei das Lösungsmittel 1,4-Dioxan ist und der Initiator 1,8-Diazabicyclo[5,4,0]undec-7-en ist.

3. Verfahren nach Anspruch 1 oder 2, wobei die Temperatur im Bereich von 10 bis 50°C liegt.

4. Verfahren nach einem der Ansprüche 1 bis 3, wobei die Konzentration des tertiären γ-Esters des α-Glutaminsäure-N-carboxyanhydrids im Bereich von 0,1 bis 0,3 M liegt.

5. Verfahren nach einem der Ansprüche 1 bis 4, wobei die Stufe b) durchgeführt wird in einer Säure, ausgewählt aus Trifluoressigsäure, Ameisensäure und Wasser/Ameisensäure-Gemischen bei einer Temperatur im Bereich von 20 bis 60°C.

6. Verfahren nach einem der Ansprüche 1 bis 5, wobei R t-Butyl ist.

7. Verfahren nach einem der Ansprüche 1 bis 6, wobei das Molekulargewicht der Poly-α-Glutaminsäure (I) im Bereich von 13.000 bis 16.000 Da liegt und der Polydispersitätsindex ≤ 1,5 ist.

## Revendications

1. Procédé pour la préparation d'un acide poly-α-glutamique de formule (I) 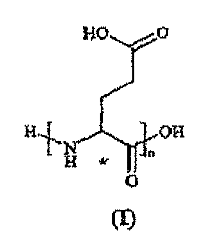 dans laquelle le symbole * indique qu'un centre chiral n est entre 60 et 310, de sorte que l'acide poly-α-glutamique a un poids moléculaire allant de 8000 à 40 000 Da
ledit procédé comprenant les étapes de :
a) polymérisation d'un ester γ tertiaire de N-carboxyanhydride d'acide α-glutamique de formule (II) 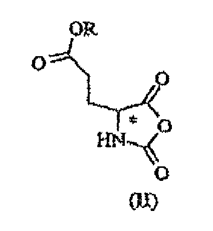 dans laquelle le symbole * est tel que défini ci-dessus et R est choisi parmi le t-butyle, le 1,1-diméthylpropyle et le 1,1-diméthylbutyle
dans l'eau ou dans un solvant organique choisi parmi : le tétrahydrofuranne, le 1,4-dioxane, le diméthylformamide, un mélange 1,4-dioxane/DMF et 1,4-dioxane/ tétrahydrofuranne avec un amorceur choisi parmi un *tert*-butoxyde de potassium, un méthoxyde de sodium, une diisopropyléthylamine, un 1,8-diazabicyclo[5,4,0]-undéc-7-ène, une diméthylaminopyridine et l'ester γ de *tert*-butyle d'un acide L-glutamique, pour obtenir un composé de formule (III) 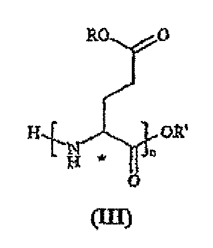 dans laquelle * et R sont tels que définis ci-dessus et R' est un atome d'hydrogène quand l'amorceur est choisi parmi une diisopropyléthylamine, un 1,8-diazabicyclo[5,4,0]-undéc-7-ène, une 4-diméthylaminopyridine, l'ester diméthylique de l'acide glutamique et l'ester γ de *tert*-butyle d'un acide L-glutamique ou R' est un groupe *t*-butyle ou méthyle quand l'amorceur est un *tert-*butoxyde de potassium et un méthoxyde de sodium, respectivement,
suivie par
b) l'hydrolyse acide des groupes ester γ et α pour obtenir un composé de formule (I).

2. Procédé selon la revendication 1, dans lequel le solvant est le 1,4-dioxane et l'amorceur est le 1,8-diazabicyclo[5,4,0]-undéc-7-ène.

3. Procédé selon les revendications 1 ou 2, dans lequel la température va de 10 à 50°C.

4. Procédé selon l'une quelconque des revendications 1 à 3, dans lequel la concentration de l'ester γ tertiaire de N-carboxyanhydride d'acide α-glutamique va de 0,1 à 0,3 M.

5. Procédé selon l'une quelconque des revendications 1 à 4, dans lequel l'étape b) est mise en oeuvre dans un acide choisi parmi l'acide trifluoroacétique, l'acide formique et des mélanges eau/acide formique à une température allant de 20 à 60 °C.

6. Procédé selon l'une quelconque des revendications 1 à 5, dans lequel R est un *t-*butyle.

7. Procédé selon l'une quelconque des revendications 1 à 6, dans lequel le poids moléculaire de l'acide poly-α-glutamique (I) va de 13 000 à 16 000 Da et son indice de polydispersité est ≤ 1,5.
